# EUROPÄISCHE PATENTSCHRIFT

(11) **EP 0 526 794 B1**
(45) Veröffentlichungstag und Bekanntmachung des Hinweises auf die Patenterteilung: **17.05.1995**
(21) Anmeldenummer: 92112414.5
(22) Anmeldetag: 21.07.1992
(51) Int. Cl.: C07C 2/86, C07C 15/16

(54) **Verfahren zur Herstellung benzylierter Aromaten**
Process for the preparation of benzylated aromatics
Procédé de préparation de composés aromatiques benzylés

(30) Priorität: 03.08.1991 DE 4125759
(43) Veröffentlichungstag der Anmeldung: 10.02.1993
(73) Patentinhaber: BAYER AG, 51368 Leverkusen (DE)
(72) Erfinder: Klein, Alfons, Dipl.-Ing., W-4000 Düsseldorf (DE); Fiege, Helmut, Dr., W-5090 Leverkusen 1 (DE); Kron, Rudolf, Dipl.-Ing., W-5303 Bornheim 2 (DE); Jelitto, Frank, Dr., W-5060 Bergisch Gladbach 2 (DE); Blazejak, Manfred, Dr., W-4000 Düsseldorf (DE)

(56) Entgegenhaltungen:
- DE-A- 3 836 780
- US-A- 4 049 733

## Beschreibung

Die Erfindung betrifft ein Verfahren zur Herstellung benzylierter Aromaten durch Umsetzung aromatischer Kohlenwasserstoffe mit Benzylalkoholen und/oder den daraus erhältlichen Dibenzylethern unter sauren Bedingungen bei erhöhter Temperatur,

Benzylierte Aromaten sind für die Verwendung als Elektroisoliermittel, Wärmeträgeröle und thermostabile Schmierstoffe ausgezeichnet geeignet, weil sie eine sehr niedrige elektrische Leitfähigkeit besitzen, chemisch inert und thermisch stabil sind.

Benzylierte Aromaten können beispielsweise durch Umsetzung aromatischer Kohlenwasserstoffe mit Formaldehyd oder durch Friedel-Crafts-Alkylierung aromatischer Kohlenwasserstoffe mit Benzylchlorid hergestellt werden. Das eine Verfahren erfordert Schutzmaßnahmen gegen die Belästigung durch Formaldehyd, das andere führt zu Korrosionsproblemen durch vom Benzylchlorid abgespaltenen Chlorwasserstoff und durch Endprodukte mit einem intolerablen Restchlorgehalt.

Aus der DE-OS 3 836 780 ist ein Verfahren zur Herstellung benzylierter Aromaten durch Umsetzung aromatischer Kohlenwasserstoffe mit Benzylalkoholen und/oder den daraus erhältlichen Dibenzylethern in Gegenwart sauer aktivierter Bleicherden bei erhöhter Temperatur bekannt. Bei der Herstellung benzylierter Aromaten mit niedrigem Benzylierungsgrad muß mit großen Verdünnungen gearbeitet werden, d.h. entweder mit einem großen Überschuß aromatischer Kohlenwasserstoffe oder in Gegenwart eines inerten Verdünnungsmittels wie Cyclohexan; es werden Mengen von 40 bis 2000 Gew.-% Verdünnungsmittel, bezogen auf umzusetzenden aromatischen Kohlenwasserstoff, empfohlen. Dieses Arbeiten in hohen Verdünnungen führt zu einer schlechten Raumausbeute, die die Wirtschaftlichkeit des Verfahrens beeinträchtigt.

Überraschenderweise wurde nun gefunden, daß sich die Raumausbeute durch Verwendung aliphatischer, gegebenenfalls perfluorierter Sulfonsäuren als Katalysatoren beträchtlich steigern läßt.

Gegenstand der Erfindung ist also ein Verfahren zur Herstellung benzylierter Aromaten der Formel 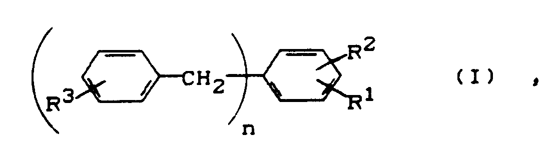
worin
- R¹ bis R³: unabhängig voneinander Wasserstoff oder C₁-C₄-Alkyl und
- n: Zahlen von 1 bis 3 bedeuten,
durch Umsetzung aromatischer Kohlenwasserstoffe der Formel 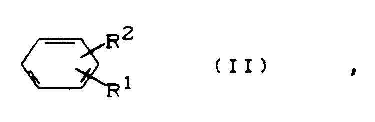
worin R¹ und R² die oben angegebene Bedeutung haben,
mit Benzylalkohol und/oder Dibenzylether der Formeln 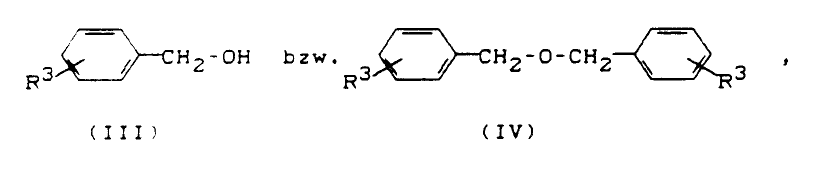
worin R³ die oben angegebene Bedeutung hat,
in Gegenwart von Säure als Katalysator bei erhöhter Temperatur, dadurch gekennzeichnet, daß als Säure eine aliphatische, gegebenenfalls perfluorierte Sulfonsäure eingesetzt wird.

Bevorzugte aromatische Kohlenwasserstoffe II umfassen beispielsweise Benzol, Toluol, Xylol, Ethylbenzol, Diethylbenzol, Isopropylbenzol und Diisopropylbenzol; besonders bevorzugt wird Toluol.

Bevorzugte Benzylalkohole III umfassen beispielsweise unsubstituierten Benzylalkohol, Methylbenzylalkohole, Ethylbenzylalkohole und Isopropylbenzylalkohole; besonders bevorzugt wird Benzylalkohol.

Bevorzugte Dibenzylether IV umfassen beispielsweise unsubstituierten Dibenzylether, Dimethyl-dibenzylether, Diethyl-dibenzylether und Diisopropyl-dibenzylether; unsubstituierter Dibenzylether wird besonders bevorzugt.

Selbstverständlich ist es auch möglich, erfingungsgemäß Gemische aus dem Benzylalkohol III und dem daraus erhältlichen Dibenzylether IV einzusetzen. Bevorzugt wird ein Gemisch aus unsubstituiertem Benzylalkohol und unsubstituiertem Dibenzylether im Molverhältnis 1:2 bis 1:5 eingesetzt.

Bevorzugte aliphatische Sulfonsäuren entsprechen der Formel 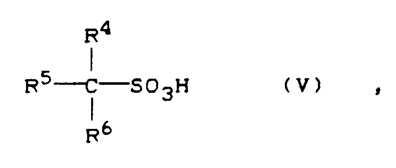
worin
- R⁴ und R⁵: unabhängig voneinander Wasserstoff oder Fluor und
- R⁶: Wasserstoff, Fluor, C₁-C₃-Alkyl oder Perfluor-C₁-C₃-alkyl bedeuten.

Geeignete aliphatische Sulfonsäuren V umfassen beispielsweise Methansulfonsäure, Ethansulfonsäure, Propansulfonsäure, Butansulfonsäure, Perfluormethansulfonsäure und Perfluorbutansulfonsäure. Bevorzugt werden Methansulfonsäure und Perfluorbutansulfonsäure; besonders bevorzugt wird Methansulfonsäure.

Das Verhältnis der Reaktionspartner II und III bzw. IV kann erfindungsgemäß in weiten Bereichen schwanken. Es hängt wesentlich von dem angestrebten Benzylierungsgrad des gebildeten benzylierten Aromaten I ab. Sollen die benzylierten Aromaten einen hohen Anteil monobenzylierten Produkts enthalten, sollte der aromatische Kohlenwasserstoff, bezogen auf Benzylierungsmittel III bzw. IV, im Überschuß eingesetzt werden. Wenn beispielsweise 90-95 Gew.-% an monobenzylierten Produkt gewünscht werden, wird ein molares Verhältnis von Aromat (II) zu Benzylierungsmittel (III) bzw. (IV), gerechnet als Benzylalkohol (III), im Bereich von 10-20:1, bevorzugt 12-16:1, eingestellt.

Bei Mehrfachbenzylierungen können im Prinzip verschiedene Reihen von Produkten entstehen, die sich durch die Art der Substitution unterscheiden, was am Beispiel der Benzylierungsprodukte des Toluols mit n = 3 erklärt werden soll: Es können Verbindungen der Formeln 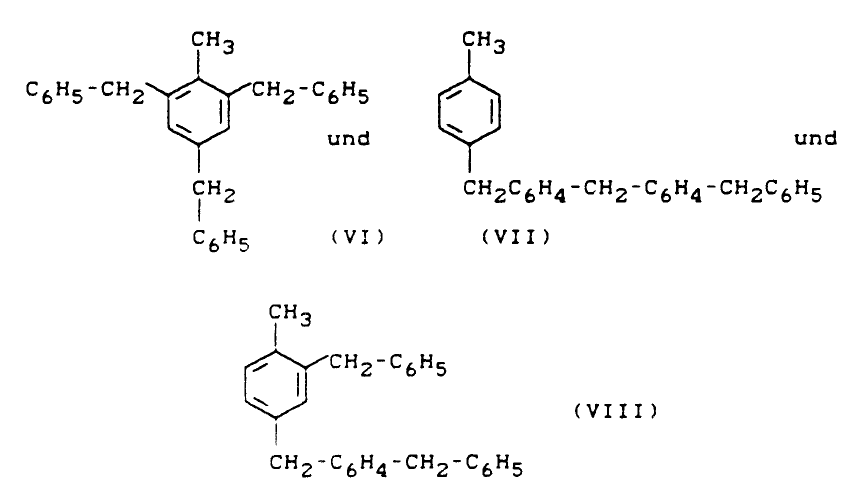
entstehen.

Wenn auch die Reaktion z.B. in Richtung der Monobenzylierungsprodukte gelenkt werden kann, so entstehen in der Praxis unter vernünftigen Reaktionsbedingungen regelmäßig auch geringe Mengen höher benzylierter Produkte, die - falls erforderlich - durch übliche Trennmethoden, wie z.B. Destillation, abgetrennt werden können. Eine gezielte Lenkung in Richtung auf Di- und Tribenzylierungsprodukte in insofern nicht möglich, als immer auch höher benzylierte Produkte mitentstehen. Wenn also n = 1,2 oder 3 bedeutet, dann ist dies im Sinne der Erfindung so zu verstehen, daß das erhaltene Produkt zu mindestens 80, vorzugsweise zu mindestens 90 Gew.-% aus solchen Produkten besteht, aber bis zu 20, vorzugsweise bis zu 10 Gew.-% anderer Benzylierungsprodukte enthalten kann.

Falls Mischungen gewünscht oder toleriert werden, die größere Mengen höher benzylierter Produkte enthalten, kann der Überschuß an aromatischem Kohlenwasserstoff II geringer ausfallen. Ein in der Elektroindustrie als Elektroisoliermittel eingesetztes Produkt mit einer Zusammensetzung von 70-85 Gew.-% Benzyltoluol, 15-30 Gew.-% Dibenzyltoluol und untergeordneten Mengen an höher benzyliertem Toluol kann beispielsweise unter Verwendung eines molaren Toluol/Benzylalkohol-Verhältnis von 4-7:1 hergestellt werden.

Bei der Verwendung oder Mitverwendung von Dibenzylethern statt der entsprechenden Benzylalkohole als Benzylierungsmittel ist für die Betrachtung der Molverhältnisse 1 Mol Dibenzylether 2 Molen Benzylalkohol gleichzusetzen.

Die aliphatische Sulfonsäure wird erfindungsgemäß in einer Menge von 0,5 bis 20 Gew.-%, bevorzugt 2,0 bis 8 Gew.-%, bezogen auf den Benzylalkohol III bzw. Dibenzylether IV eingesetzt. Sie kann in reiner Form, aber auch als wäßrige Lösung verwendet werden, beispielsweise in der 70 gew.-%igen wäßrigen Handelsform der Methansulfonsäure.

Das erfindungsgemäße Verfahren kann bei einer Temperatur von 80 bis 160°C, bevorzugt bei 90 bis 150°C und besonders bevorzugt bei 100 bis 140°C durchgeführt werden. In ganz besonders bevorzugter Weise wird bei der Siedetemperatur des Reaktionsgemisches gearbeitet.

Im erfindungsgemäßen Verfahren wird Reaktionswasser gebildet. Das Reaktionswasser kann grundsätzlich im Reaktionsgemisch verbleiben und erst bei der Aufarbeitung abgetrennt werden. Dabei ist es auch möglich, in einem Druckgefäß zu arbeiten, um Temperaturen oberhalb des Siedepunktes des Reaktionsgemisches zu erreichen. Damit wird eine Erhöhung der Reaktionsgeschwindigkeit erreicht.

In einer bevorzugten Ausführungsform wird das Reaktionswasser jedoch bereits während der Kondensation destillativ aus dem Reaktionsgemisch entfernt und ausgeschleust. Hierbei wird die Tatsache in vorteilhafter Weise ausgenutzt, daß viele aromatische Kohlenwasserstoffe II mit Wasser ein Azeotrop bilden, welches nach der Kondensation außerhalb des Reaktionsgemisches in einem Wasserabscheider wieder in Aromat und Wasser getrennt wird. Der aromatische Kohlenwasserstoff kann wieder in das Reaktionsgemisch zurückgeführt werden, während das ausgeschleuste Wasser als ein Maß für den Fortschritt der Reaktion dient.

Es ist ein besonderer Vorteil des erfindungsgemäßen Verfahrens, daß der Überschuß an aromatischen Kohlenwasserstoffen II sehr niedrig gehalten werden kann. Gegenüber bekannten Verfahren, die beispielsweise aktivierte Bleicherde als Katalysator verwenden, wird dieser Vorteil besonders deutlich: Das erfindungsgemäße Verfahren ist mit einer deutlich erhöhten Raumausbeute verbunden.

Das erfindungsgemäße Verfahren kann in vielfältiger Weise ausgeführt werden. So ist es beispielsweise in der bereits angegebenen Weise möglich, alle Ausgangsmaterialien einschließlich der aliphatischen Sulfonsäure vorzulegen und auf die gewünschte Reaktionstemperatur zu erhitzen. Des weiteren ist es möglich, den aromatischen Kohlenwasserstoff II und die aliphatische Sulfonsäure vorzulegen und erst dann den Benzylalkohol und/oder Dibenzylether zuzugeben. Eine solche Zugabe erfolgt in bevorzugter Weise nach Maßgabe der Wasserentwicklung, wozu in einem solchen bevorzugten Fall das Reaktionswasser in der beschriebenen Weise aus dem Reaktionsgemisch bereits während der Reaktion abgetrennt wird.

Für den Fall, daß eine Arbeitsweise gewählt wird, in der das Reaktionswasser im Gemisch verbleibt, kann die Aufarbeitung in der Weise erfolgen, daß der Katalysator, der sich in dem gebildeten Wasser auflöst, mit diesem gemeinsam von der organischen Schicht abgetrennt wird, Die gebrauchte, wasserhaltige Sulfonsäure kann nach Entwässerung wieder verwendet werden. Diese Entwässerung kann selbstverständlich auch durch azeotrope Destillation mit dem aromatischen Kohlenwasserstoff II erfolgen. Die Isolierung des gebildeten benzylierten Aromaten I aus der organischen Phase kann nach einer alkalischen Wäsche beispielsweise durch eine Destillation erfolgen.

Wird nach der bevorzugten Weise gearbeitet, bei der das Reaktionswasser bereits während der Kondensation destillativ aus dem Reaktionsgemisch entfernt wird, kann die Aufarbeitung ähnlich erfolgen. Die möglichst quantitative Abtrennung der gebrauchten aliphatischen Sulfonsäure V kann beispielsweise durch Zugabe von Wasser erreicht werden. Dadurch entsteht eine verdünnte Säure, die sich in dieser Form leicht von der organischen Schicht abtrennen läßt. Die gebrauchte wasserhaltige Sulfonsäure V kann entweder in dieser Form oder nach Entwässerung wieder verwendet werden. Die Isolierung des gebildeten benzylierten Aromaten I aus der organischen Phase kann auch hier nach einer alkalischen Wäsche beispielweise durch Destillation erfolgen.

Die Prozentangabe der nachfolgenden Beispiele beziehen sich jeweils auf das Gewicht.

### Beispiele

### Beispiel 1

In einem 1-l-Dreihalskolben füllt man 250,3 g Toluol und 10 g 70%ige wäßrige Methansulfonsäure-Lösung. Sobald die Sumpftemperatur 95°C erreicht hat, d.h. bei Siedebeginn, wird innerhalb von 3 Stunden 158,4 g Dibenzylether eingetropft. Wasser wird azeotrop entfernt. Im Laufe der Umsetzung steigt die Sumpftemperatur von ca. 95°C auf ca. 136°C. Nach dem Eintropfen läßt man noch 0,5 Stunde unter Rückfluß sieden. Danach ist die Reaktion beendet. Das Reaktionsgemisch wird anschließend bei 80°C mit 15 ml Wasser versetzt und kurz aufgekocht. Die verdünnte Methansulfonsäure setzt sich ab und kann glatt abgetrennt werden. Sie läßt sich rückführen.

Die organische Phase wird nach Zugabe von 2 g 45%iger Natronlauge über eine Destillationsbrücke destilliert.

Hauptfraktion: Kp₁ = 80 - 195°C (235 g).

Der Destillationsrückstand wiegt 13,4 g.

Zusammensetzung der Hauptfraktion:

| | |
|---|---|
| Benzyltoluol: | 73,9% |
| Dibenzyltoluol: | 24,0% |
| Polybenzyltoluole: | 2,1% |

### Beispiel 2

In einen emaillierten Autoklaven füllt man 250,3 g Toluol und 30 g Methansulfonsäure. Unter Rühren bei 100°C werden 215 g Dibenzylether eingetropft. Dann wird der Autoklav geschlossen und 6 Stunden bei 145°C gerührt. Nach der Überführung des Reaktionsgemisches in einen Scheidetrichter setzt sich die wasserhaltige Methansulfonsäure ab und kann abgetrennt werden. Sie läßt sich rückführen. Die organische Phase wird nach Zugabe von 2 g 45%iger Natronlauge über eine Destillationsbrücke destilliert.

Hauptfraktion: Kp₁ = 80 - 195°C (307,8 g).

Der Destillationsrückstand wiegt 15,1 g.

Zusammensetzung der Haupfraktion:

| | |
|---|---|
| Benzyltoluol: | 73,8% |
| Dibenzyltoluol: | 24,1% |
| Polybenzyltoluole: | 2,1% |

### Vergleichsbeispiel 1

Zu 250,3 g Toluol und 2,5 g säurebehandelter Bleicherde werden bei Rückflußtemperatur innerhalb von 3 Stunden 158,4 g Dibenzylether zugetropft und das entstandene Reaktionswasser azeotrop abdestilliert. Man läßt 1/2 Stunde unter Rückfluß nachreagieren und saugt dann vom Katalysator ab. Das Filtrat wird nach Zugabe von 2 g 45%iger Natronlauge über eine Destillationsbrücke destilliert.

Hauptfraktion: Kp₁ = 80 - 195°C (151,6 g).

Der Destillationsrückstand wiegt 40,1 g.

Zusammensetzung der Hauptfraktion:

| | |
|---|---|
| Benzyltoluol: | 67,9% |
| Dibenzyltoluol: | 30,1% |
| Polybenzyltoluole: | 2,0% |

### Vergleichsbeispiel 2

Zu 531 g Toluol und 2,5 g säurebehandelter Bleicherde werden bei Rückflußtemperatur innerhalb von 3 Stunden 158,4 g Dibenzylether zugetropft und das enstandene Reaktionswasser azeotrop abdestilliert Man läßt 1/2 Stunde unter Rückfluß nachreagieren und saugt dann vom Katalysator ab. Das Filtrat wird nach Zugabe von 2 g 45%iger Natronlauge über eine Destillationsbrücke destilliert.

Hauptfraktion: Kp₁ = 80 - 195°C (208,5 g).

Der Destillationsrückstand wiegt 16,6 g.

Zusammensetzung der Hauptfraktion:

| | |
|---|---|
| Benzyltoluol: | 74,8% |
| Dibenzyltoluol: | 23,0% |
| Polybenzyltoluole: | 2,2% |

## Patentansprüche

1. Verfahren zur Herstellung benzylierter Aromaten der Formel 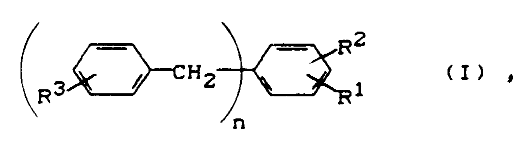 worin
R¹ bis R³ unabhängig voneinander Wasserstoff oder C₁-C₄-Alkyl und
n Zahlen von 1 bis 3 bedeuten,
durch Umsetzung aromatischer Kohlenwasserstoffe der Formel 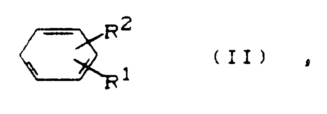 worin R¹ und R² die oben angegebene Bedeutung haben,
mit Benzylalkohol und/oder Dibenzylether der Formeln 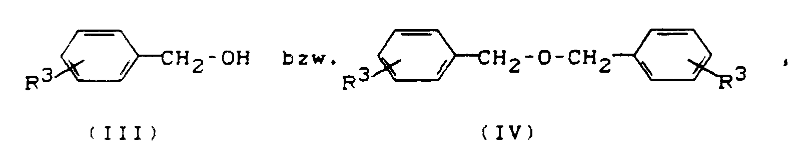 worin R³ die oben angegebene Bedeutung hat,
in Gegenwart von Säure als Katalysator bei erhöhter Temperatur, dadurch gekennzeichnet, daß als Säure eine aliphatische Sulfonsäure eingesetzt wird.

2. Verfahren nach Anspruch 1, wonach der aromatische Kohlenwasserstoff II aus der Reihe Benzol, Toluol, Xylol, Ethylbenzol, Diethylbenzol, Isopropylbenzol und Diisopropylbenzol ausgewählt wird.

3. Verfahren nach Anspruch 1, wonach der Benzylalkohol III aus unsubstituiertem Benzylalkohol, Methylbenzylalkoholen, Ethylbenzylalkoholen und Isopropylbenzylakoholen ausgewählt wird.

4. Verfahren nach Anspruch 1, wonach der Dibenzylether IV aus unsubstutuiertem Dibenzylether, Dimethyldibenzylethern, Diethyl-dibenzylethern und Diisopropyl-dibenzylethern ausgewählt wird.

5. Verfahren nach Anspruch 1, wonach die Sulfonsäure aus Verbindungen der Formel 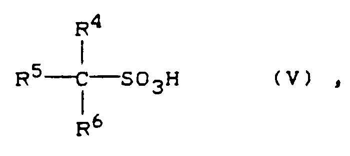 worin
R⁴ und R⁵ unabhängig voneinander Wasserstoff oder Fluor und
R⁶ Wasserstoff, Fluor, C₁-C₃-Alkyl oder Perfluor-C₁-C₃-Alkyl bedeuten, ausgewählt wird.

6. Verfahren nach Anspruch 1, wonach die Sulfonsäure aus den Verbindungen Methansulfonsäure, Ethansulfonsäure, Propansulfonsäure, Butansulfonsäure, Perfluormethansulfonsäure und Perfluorbutansulfonsäure ausgewählt wird.

7. Verfahren nach Anspruch 1, wonach die Sulfonsäure in Mengen von 0,5 bis 20 Gew.-%, bezogen auf Benzylalkohol III bzw. Dibenzylether IV, eingesetzt wird.

8. Verfahren nach Anspruch 1, wonach die Sulfonsäure in Mengen von 2 bis 8 Gew.-%, bezogen auf Benzylalkohol III bzw. Dibenzylether IV, eingesetzt wird.

9. Verfahren nach Anspruch 1, wonach die Umsetzung bei einer Temperatur von 80 bis 160°C durchgeführt wird.

## Claims

1. Process for the preparation of benzylated aromatics of the formula 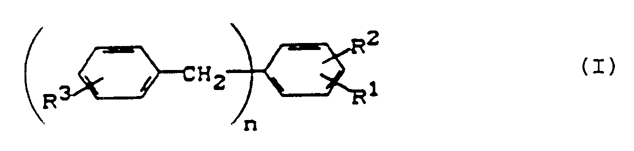 wherein
R¹ to R³ independently of one another denote hydrogen or C₁-C₄-alkyl and
n denotes numbers from 1 to 3,
by reaction of aromatic hydrocarbons of the formula 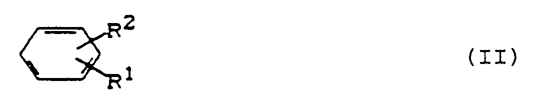 wherein R¹ and R² have the abovementioned meaning,
with benzyl alcohol and/or dibenzyl ether of the formulae 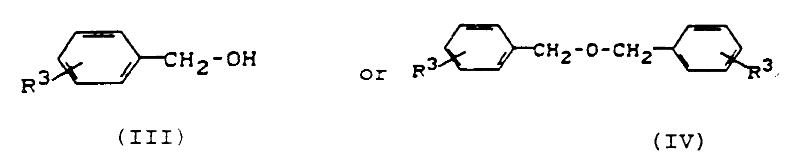 wherein R³ has the abovementioned meaning,
in the presence of acid as the catalyst at elevated temperature, characterised in that an aliphatic sulphonic acid is employed as the acid.

2. Process according to Claim 1, in which the aromatic hydrocarbon II is chosen from the series comprising benzene, toluene, xylene, ethylbenzene, diethylbenzene, isopropylbenzene and diisopropylbenzene.

3. Process according to Claim 1, in which the benzyl alcohol III is chosen from unsubstituted benzyl alcohol, methylbenzyl alcohols, ethylbenzyl alcohols and isopropylbenzyl alcohols.

4. Process according to Claim 1, in which the dibenzyl ether IV is chosen from unsubstituted dibenzyl ether, dimethyl-dibenzyl ethers, diethyl-dibenzyl ethers and diisopropyl-dibenzyl ethers.

5. Process according to Claim 1, in which the sulphonic acid is chosen from compounds of the formula 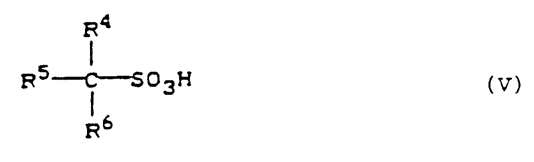 wherein
R⁴ and R⁵ independently of one another denote hydrogen or fluorine and
R⁶ denotes hydrogen, fluorine, C₁-C₃-alkyl or perfluoro-C₁-C₃-alkyl.

6. Process according to Claim 1, in which the sulphonic acid is chosen from the compounds methanesulphonic acid, ethanesulphonic acid, propanesulphonic acid, butanesulphonic acid, perfluoromethanesulphonic acid and perfluorobutanesulphonic acid.

7. Process according to Claim 1, in which the sulphonic acid is employed in amounts of 0.5 to 20% by weight, based on the benzyl alcohol III or dibenzyl ether IV.

8. Process according to Claim 1, in which the sulphonic acid is employed in amounts of 2 to 8% by weight, based on the benzyl alcohol III or dibenzyl ether IV.

9. Process according to Claim 1, in which the reaction is carried out at a temperature from 80 to 160°C.

## Revendications

1. Procédé de préparation de composés aromatiques benzylés de formule 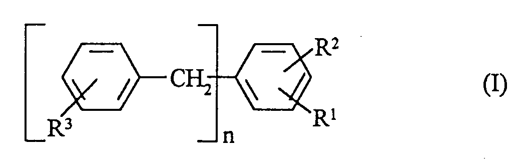 dans laquelle R¹ à R³ sont indépendamment les uns des autres un hydrogène ou un alkyle en C₁-C₄, et
n représente un nombre de 1 à 3,
par réaction d'hydrocarbures aromatiques de formule 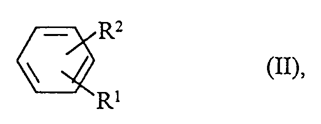 dans laquelle R¹ et R² ont la signification donnée ci-dessus,
avec un alcool benzylique et/ou un éther dibenzylique ayant respectivement les formules 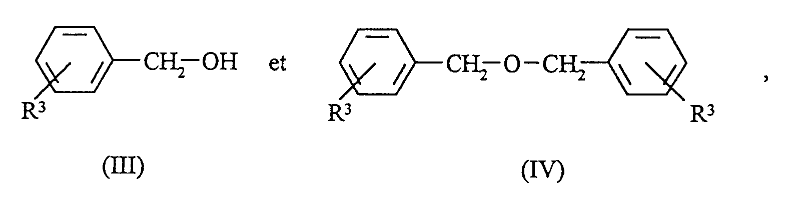 dans lesquelles R³ a la signification donnée ci-dessus,
en présence d'un acide comme catalyseur, à température élevée, caractérisé en ce que l'on utilise comme acide un acide sulfonique aliphatique.

2. Procédé selon la revendication 1, dans lequel l'hydrocarbure aromatique II est choisi dans la série du benzène, du toluène, du xylène, de l'éthylbenzène, du diéthylbenzène, de l'isopropylbenzène et du diisopropylbenzène.

3. Procédé selon la revendication 1, dans lequel l'alcool benzylique III est choisi parmi l'alcool benzylique non substitué, les alcools méthylbenzyliques, les alcools éthylbenzyliques et les alcools isopropylbenzyliques.

4. Procédé selon la revendication 1, dans lequel l'éther dibenzylique IV est choisi parmi l'éther dibenzylique non substitué, les éthers diméthyldibenzyliques, les éthers diéthyldibenzyliques et les éthers diisopropyldibenzyliques.

5. Procédé selon la revendication 1, dans lequel l'acide sulfonique est choisi parmi les composés de formule 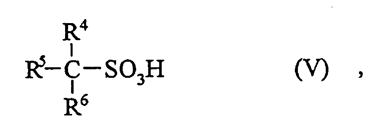 dans laquelle
R⁴ et R⁵ sont indépendamment l'un de l'autre un hydrogène ou un fluor, et
R⁶ représente un hydrogène, un fluor, un alkyle en C₁-C₃ ou un perfluoroalkyle en C₁-C₃.

6. Procédé selon la revendication 1, dans lequel l'acide sulfonique est choisi parmi l'acide méthanesulfonique, l'acide éthanesulfonique, l'acide propanesulfonique, l'acide butanesulfonique, l'acide perfluorométhanesulfonique et l'acide perfluorobutanesulfonique.

7. Procédé selon la revendication 1, dans lequel l'acide sulfonique est utilisé en des quantités de 0,5 à 20 % en masse par rapport à l'alcool benzylique III ou à l'éther dibenzylique IV.

8. Procédé selon la revendication 1, dans lequel l'acide sulfonique est utilisé en des quantités de 2 à 8 % en masse par rapport à l'alcool benzylique III ou à l'éther dibenzylique IV.

9. Procédé selon la revendication 1, dans lequel la réaction s'effectue à une température de 80 à 160°C.
